# EUROPÄISCHE PATENTSCHRIFT

(11) **EP 2 569 028 B1**
(45) Veröffentlichungstag und Bekanntmachung des Hinweises auf die Patenterteilung: **23.09.2015**
(21) Anmeldenummer: 11732360.0
(22) Anmeldetag: 13.05.2011
(51) Int. Cl.: A61M 1/16

(54) **SYSTEM ZUR HERSTELLUNG VON REINSTWASSER IN DER DIALYSE**
SYSTEM FOR PRODUCING ULTRA-PURE WATER IN DIALYSIS
SYSTÈME DE PRODUCTION D'EAU ULTRAPURE EN DIALYSE

(30) Priorität: 14.05.2010 DE 202010006823 U
(43) Veröffentlichungstag der Anmeldung: 20.03.2013
(73) Patentinhaber: Fresenius Medical Care Deutschland GmbH, 61352 Bad Homburg (DE)
(72) Erfinder: KIRSCHNER, Ulrich, 61350 Bad Homburg (DE)
(74) Vertreter: Laufhütte, Dieter
(86) Internationale Anmeldenummer: PCT/EP2011/002395
(87) Internationale Veröffentlichungsnummer: WO 2011/141186

(56) Entgegenhaltungen:
- EP-A2- 0 352 779
- DE-A1- 10 262 036
- DE-U1- 20 116 498
- US-A1- 2004 182 773
- US-A1- 2006 241 543

## Beschreibung

Die vorliegende Erfindung betrifft ein System zur Herstellung von Reinstwasser in der Dialyse mit einer Umkehr-Osmose-Anlage, welche einen Zulauf für Frischwasser und einen Ablauf für Reinstwasser aufweist, und mit einer Vorrichtung zum Einbringen von Ozon.

Da beim Betrieb von Dialysemaschinen zur Herstellung von Dialysat große Mengen an Reinstwasser benötigt werden, wird dieses in Dialysezentren oftmals durch eine fest installierte Vorrichtung zur Herstellung von Reinstwasser bereitgestellt. An eine solche Vorrichtung können dann ein oder mehrere Dialysegeräte angeschlossen werden, welche von der Vorrichtung Reinstwasser zur Herstellung von Dialysat beziehen. Das erfindungsgemäße System zur Herstellung von Reinstwasser umfasst dabei eine solche Vorrichtung zur Herstellung von Reinstwasser. Gegebenenfalls kann das System dabei weiterhin ein oder mehrere an die Vorrichtung angeschlossene Dialysegeräte umfassen.

Vorrichtungen zum Einbringen von Ozon in einen Flüssigkeitsstrom werden eingesetzt, um eine Verkeimung der entsprechenden Anlagen zu verhindern. Ozon stellt ein sehr starkes Oxidationsmittel dar, welches alle bekannten mikrobiologischen Lebensformen im Wasser - auch Sporen und Pilze - sicher abtötet. Ozon zersetzt dieses organische Material dabei bis zu Kohlendioxid und Wasser, und zerfällt nach einiger Zeit ohne Reaktionspartner im Wasser von allein wieder zu Sauerstoff. Dabei ist es bekannt, das im Wasser verbleibende Restozon durch den Einsatz von UV-Licht zu vernichten. Dies hat den Nachteil, dass am Vernichtungsort immer elektrische Energie benötigt wird. Zudem ist die Technik relativ aufwendig, so dass im Reinstwasserbereich von Dialysesystemen auf eine Ozonbehandlung bisher weitgehend verzichtet wurde.

Aus der DE 102 62 036 A1 ist eine Reinstwasserversorgungsanlage für Dialysegeräte mit einer Permeatringleitung bekannt, in welcher stromaufwärts einer Zirkulationspumpe eine Komponente zur physikalischen Desinfektion vorgesehen ist, mit welcher der Permeatinhalt der Permeatringleitung während der Stillstandeszeiten des Dialysebetriebs desinfiziert werden kann. Als Möglichkeit Komponente zur physikalischen Desinfektion kann ein Ozon-Reaktor vorgesehen sein.

Aus der US 2004/0182773 A1 ist es bekannt, eine Dialysemaschine über ozoniertes Wasser zu desinfizieren.

Aus der DE 201 16 498 U1 ist es bekannt, dem bei der Behandlung eingesetzten Dialysat Ozon in einer solchen Menge beizufügen, dass die Patienten 3 bis 6 ppm Ozon im Blut haben. Dem wird eine langfristige sterilisierende Wirkung auf den Körper zugeschrieben.

Aufgabe der vorliegenden Erfindung ist es, eine einfache und kostengünstige Möglichkeit zur Verhinderung der Verkeimung von Reinstwasseranlagen in der Dialyse zur Verfügung zu stellen.

Diese Aufgabe wird erfindungsgemäß durch ein System gemäß Anspruch 1 gelöst. Das erfindungsgemäße System zur Herstellung von Reinstwasser in der Dialyse umfasst dabei eine Umkehr-Osmose-Anlage, welche einen Zulauf für Frischwasser und einen Ablauf für Reinstwasser aufweist, und eine Vorrichtung zum Einbringen von Ozon. Erfindungsgemäß ist dabei ein Ozon-Filter vorgesehen, welcher stromabwärts des Ablaufs der Umkehr-Osmose-Anlage angeordnet ist und von der Vorrichtung zum Einbringen von Ozon eingebrachtes Ozon wieder entfernt. Da Ozon sehr reaktionsfähig ist, kann das Gas an der großen Oberfläche des Ozonfilters adsorbiert und durch den Zerfall zu Sauerstoff unschädlich gemacht werden. Gleichzeitig hält das im Ozonfilter adsorbierte Ozon diesen steril. Ein weiterer Vorteil des Einsatzes von Ozon ist dessen Eigenschaft, Endotoxine zu vernichten, was für den Einsatz in der Dialyse besonders vorteilhaft ist. Durch die Anordnung des Ozon-Filters stromabwärts der Umkehr-Osmose-Anlage kann dieser das von der Umkehr-Osmose-Anlage erzeugte Reinstwasser oder ein dieses Reinstwasser enthaltendes Dialysat wieder von Ozon befreien. Durch die vorliegende Erfindung kann Ozon so auch im Reinstwasserbereich eingesetzt werden, um den Keimbefall einzudämmen. Unter einer Anordnung stromabwärts des Ablaufs der Umkehr-Osmose-Anlage wird dabei verstanden, dass von der Umkehr-Osmose-Anlage erzeugtes Reinstwasser im Betrieb des Systems durch den Ozonfilter fließt.

Vorteilhafterweise wird als Ozonfilter dabei ein Aktivkohlefilter eingesetzt. Dabei hat sich herausgestellt, dass handelsübliche Aktivkohlewasserfilter eine ausreichende Ozonzerstörung aufweisen. Hierdurch kann ein kostengünstiges Standardelement zur Vernichtung der Ozonrestmenge zersetzt werden.

Als Vorrichtung zum Einbringen von Ozon wird dabei vorteilhafterweise eine Anlage mit elektrolytischer Ozonerzeugung eingesetzt. Zumindest ein Teilstrom des zu behandelnden Wassers wird dabei über eine Gleichspannungszelle geleitet, in welcher das Wasser zu Sauerstoff und Wasserstoff zerlegt wird. Ein Teil des gebildeten Sauerstoffs wird mit Hilfe eines Katalysators zu Ozon umgewandelt. Vorteilhafterweise arbeitet diese Anlage dabei mit einer festen Polymerelektrolytmembran (PAM) als Katalysator. Das gebildete Ozon löst sich direkt in dem die Zelle durchströmenden Wasser. Die freien Gase werden über eine Entlüftungsleitung aus der Zelle abgeleitet. Der Wasserstrom mit dem gelösten Ozon wird dann zurückgeleitet und mit dem übrigen Strom vermischt. Alternativ zu einer elektrolytischen Anlage kann jedoch z.B. auch eine Ozonerzeugung mittels elektrischer Entladung aus gasförmigem Sauerstoff erfolgen.

Bei dem erfindungsgemäßen System zur Herstellung von Reinstwasser ist die Vorrichtung zum Einbringen von Ozon Teil der Vorrichtung zur Herstellung von Reinstwasser und damit im Bereich vor den Anschlüssen für das oder die Dialysegeräte angeordnet. Vorteilhafterweise ist die Vorrichtung zum Einbringen von Ozon dabei stromabwärts des Ablaufs der Umkehr-Osmose-Anlage angeordnet, so dass sie das Ozon in Reinstwasser einbringt, welches von der Umkehr-Osmose-Anlage zur Verfügung gestellt wurde.

Die Anordnung der Vorrichtung zum Einbringen von Ozon im Reinstwasserbereich des Systems ermöglicht eine sichere Verhinderung der Verkeimung der Anlage. Der stromabwärts der Vorrichtung zum Einbringen von Ozon angeordnete Ozonfilter sorgt dabei zuverlässig für die Vernichtung der Ozonrestmengen, so dass eine Gefährdung des Dialysepatienten durch das Ozon vermieden werden kann.

Die vorliegende Erfindung kann dabei insbesondere bei solchen Systemen zur Herstellung von Reinstwasser eingesetzt werden, welche eine Verteilungsringleitung aufweisen, in welcher Reinstwasser vom Ablauf der Umkehr-Osmose-Anlage aus an Anschlussbereichen für Dialysegeräte vorbei in einem Kreislauf gepumpt wird.

Gegebenenfalls kann dabei vorgesehen sein, dass das bereits durch die Umkehr-Osmose-Anlage gereinigte Reinstwasser nach Durchlaufen des Kreislaufes nochmals durch die Umkehr-Osmose-Anlage hindurchfließt. Alternativ kann die Verteilungsringleitung auch komplett nach der Umkehr-Osmose-Anlage angeordnet sein. Z. B. kann dabei ein Tank in der Verteilungsringleitung vorgesehen sein, in welchen das von der Umkehr-Osmose-Anlage bereitgestellte Reinstwasser geleitet wird. Als stromabwärts des Ablaufs des Umkehr-Osmose-Anlage wird dabei erfindungsgemäß jeder Punkt des Systems betrachtet, welcher im Betrieb von aus dem Ablauf der Umkehr-Osmose-Anlage fließendem Reinstwasser erreicht wird. Der Bereich stromabwärts der Umkehr-Osmose-Anlage umfasst daher beim Einsatz einer Versorgungsringleitung jeden Punkt dieser Leitung.

Ist ein solcher Verteilungsring vorgesehen, ist vorteilhafterweise auch die Vorrichtung von Einbringen von Ozon in dieser Verteilungsringleitung angeordnet.

Vorteilhafterweise kann vorgesehen sein, dass auch der Ozon-Filter in der Verteilungsringleitung angeordnet ist. Insbesondere weist die Verteilungsringleitung dabei einen Abschnitt mit zwei parallelen Leitungen auf, durch welche der Flüssigkeitsstrom durch Schalten entsprechender Ventile alternativ geführt werden kann, wobei nur in einer der beiden parallelen Leitungen ein Ozonfilter angeordnet ist.

Es sind jedoch auch Anordnungen des Ozonfilters in anderen Bereichen möglich, welche weiter unten noch näher beschrieben werden.

Bei einem System zur Herstellung von Reinstwasser in der Dialyse können neben einer Verteilungsringleitung, welche einen Primärkreislauf darstellt, gegebenenfalls auch sekundäre Kreisläufe zum Anschluß einzelner Dialysegeräte vorgesehen sein. Üblicherweise zweigen diese Sekundärkreisläufe vom Primärkreislauf ab.

Dabei kann der Ozonfilter bzw. können die Ozonfilter auch im Bereich eines solchen Sekundärkreislaufes angeordnet sein. Vorteilhafterweise ist in diesem Fall jeder Sekundärkreislauf mit einem Ozon-Filter ausgestattet. Ggf. kann der Ozonfilter bzw. können die Ozonfilter auch zusätzlich zu einem Ozonfilter im Bereich der Verteilungsringleitung in einem Sekundärkreislauf eingebracht werden.

Alternativ kann der Ozon-Filter jedoch auch im Bereich der Anschlussstellen oder des bzw. der Dialysegeräte angeordnet sein. Dies hat den Vorteil, dass ein größerer Teil des Reinstwasserbereichs durch die Ozon-Behandlung vor Verkeimung geschützt werden kann. Insbesondere kann dabei vorgesehen sein, dass das zum Betrieb des oder der Dialysegeräte an den Anschlüssen zur Verfügung gestellte Reinstwasser Ozon enthält. Der Ozonfilter kann dabei direkt am Einleitungsort des Reinstwassers in die Dialysemaschine oder auch an einem geeigneten Ort in der Dialysemaschine oder direkt am Wasser-/Dialysateinlass des Dialysefilters angeordnet sein.

Bei dem erfindungsgemäßen System kann die Behandlung des Wassers mit Ozon kontinuierlich während des Betriebs erfolgen, da durch den Ozon-Filter im Reinstwasserbereich eine problemlose und kostengünstige Vernichtung von Ozon-Restmengen möglich ist. Vorteilhafterweise weist das System dabei eine Steuerung auf, welche die Vorrichtung zum Einbringen von Ozon so ansteuert, dass diese während des Betriebes des Systems Ozon erzeugt. Insbesondere wird das Ozon dabei kontinuierlich erzeugt. Damit kann erfindungsgemäß das Wasser auch dann mit Ozon behandelt werden, wenn gerade Reinstwasser zu einem oder mehreren angeschlossenen Dialysegeräten geleitet wird. Insbesondere kann das Ozon dabei auch eingebracht werden, während die Dialysegeräte eine Dialysebehandlung durchführen.

Selbst wenn Ozon in extrem geringen Mengen eventuell übrig bleiben sollte, würde dieses mit den in der Dialysierflüssigkeit enthaltenen Zuckern reagieren. Daher kann eine Zytotoxizität komplett ausgeschlossen werden, auch wenn keine 100 % Ozon-Elimination durch die Ozon-Filter erreicht wird und z. B. Ozon noch in Mengen vorliegt, wie sie für den Trinkwasserbereich zulässig.

Alternativ kann die Ozonisierung jedoch auch nur in regelmäßigen Abständen erfolgen, wobei die Ozonisierung dann vorteilhafterweise zu einem Zeitpunkt erfolgt, in welchem die Dialysegeräte kein Reinstwasser entnehmen. Die Ozonisierung kann dabei z.B. 1-5 mal pro Woche erfolgen. Der Ozongenerator liegt dabei vorteilhafterweise in einem speziellen Ozonisierungskreislauf, damit die Ozonisierung jederzeit gestoppt werden kann.

Dabei ist vorteilhafterweise eine Ventilanordnung vorgesehen, durch welche der Reinstwasserstrom durch den Primärkreislauf alternativ durch den Ozon-Filter oder eine parallele Leitung ohne Ozon-Filter geführt werden kann. Der Ozon-Filter wird dann vorteilhafterweise nur dann aktiv geschaltet, wenn die Ozonisierung abgeschlossen ist. Durch das anschließende Zirkulieren des Reinstwassers durch den Ozon-Filter des Primärkreislaufs wird das Ozon nun aus dem Reinstwasser entfernt.

Ggf. nach einer gewissen Zirkulationszeit kann dem System daher wieder Reinstwasser für die Dialysegeräte entnommen werden. Vorteilhafterweise stromabwärts des Primärkreislaufs permanent angeordnete weitere Ozon-Filter können dann ggf. übrig gebliebenes Ozon binden.

Wie bereits oben ausgeführt, umfasst das System zur Herstellung von Reinstwasser eine Vorrichtung zur Herstellung von Reinstwasser, kann aber weiterhin auch die angeschlossenen Dialysemaschinen mit umfassen. Umfasst das System dabei lediglich die Vorrichtung zur Herstellung von Reinstwasser, so ist wie oben beschrieben der Ozon-Filter im Bereich der Vorrichtung angeordnet. Umfasst das System weiterhin auch Dialysemaschinen, so kann der Ozon-Filter bzw. können die Ozon-Filter auch im Bereich dieser Dialysemaschinen angeordnet seien.

Das erfindungsgemäße System kann weiterhin eine Vorrichtung zur Herstellung von Reinstwasser in der Dialyse mit einer Umkehr-Osmose-Anlage, welche einen Zulauf für Frischwasser und einen Ablauf für Reinstwasser aufweist, eine Vorrichtung zum Einbringen von Ozon und ein oder mehrere Anschlüsse für Dialysegeräte umfassen. Dabei ist vorgesehen, dass die Vorrichtung zum Einbringen von Ozon stromabwärts des Ablaufs der Umkehr-Osmose-Anlage angeordnet ist und Ozon in das aus dem Ablauf der Umkehr-Osmose-Anlage fließende Reinstwasser einbringt. Hierdurch ist eine sichere Entkeimung bzw. ein sicherer Schutz vor Keimwachstum des Reinstwasserbereiches gewährleistet.

Vorteilhafterweise wird eine solche Vorrichtung so aufgebaut sein, wie dies oben hinsichtlich des Systems zur Herstellung von Reinstwasser dargestellt wurde. Die erfindungsgemäße Vorrichtung ist jedoch auch unabhängig von einem solchen System erfindungsgemäß einsetzbar.

Das erfindungsgemäße System kann weiterhin eine Vorrichtung zur Herstellung von Reinstwasser in der Dialyse mit einer Umkehr-Osmose-Anlage, welch einen Zulauf für Frischwasser und ein Ablauf für Reinstwasser aufweist, mit einer Vorrichtung zum Einbringen von Ozon und mit einem oder mehreren Anschlüssen für Dialysegeräte umfassen. Dabei ist vorgesehen, dass das zum Betrieb des oder der Dialysegeräte an den Anschlüssen zur Verfügung gestellte Reinstwasser Ozon enthält.

Vorteilhafterweise wird eine solche Vorrichtung so aufgebaut sein, wie dies oben hinsichtlich des Systems zur Herstellung von Reinstwasser dargestellt wurde. Insbesondere kommt eine solche Vorrichtung dabei in einem System, wie es oben dargestellt wurde, zum Einsatz. Insbesondere ist dabei vorteilhafterweise ein Ozon-Filter im Bereich des bzw. der Dialysegeräts angeordnet.

Bei den erfindungsgemäßen Vorrichtungen kann es sich dabei um fest installierte Einheiten handeln, welche z.B. in einem Dialysezentrum die Dialysegeräte mit Reistwasser versorgen.

Das erfindungsgemäße System kann weiterhin ein Dialysegerät umfassen. Das Dialysegerät umfasst dabei einen Anschluss zur Verbindung mit einer Vorrichtung zur Herstellung von Reinstwasser, von welchem das Dialysegerät Reinstwasser bezieht. Vorteilhafterweise weist das Dialysegerät dabei einen Ozon-Filter auf, um Restozonmengen im Reinstwasser zu vernichten.

Der Ozon-Filter kann im Bereich des Anschlusses des Dialysegeräts, in einem stromabwärts dieses Anschlusses und stromaufwärts des Dialysateinlaufs eines Dialysefilters angeordneten Bereich oder am Dialysateinlauf des Dialysefilters angeordnet sein. Vorteilhafterweise handelt es sich bei dem Ozon-Filter dabei, wie bereits oben dargestellt, um einen Aktivkohlefilter.

Das erfindungsgemäße System kann weiterhin ein Dialysatschlauchset und/oder einen Dialysefilter umfassen. Das Dialysatschlauchset und/oder der Dialysefilter umfassen dabei einen Ozon-Filter, welcher in einem stromaufwärts des Dialysateinlaufs des Dialysefilters angeordneten Bereich oder direkt am Dialysateinlauf des Dialysefilters angeordnet ist. Bei dem Ozon-Filter handelt es sich dabei, wie oben dargestellt, vorteilhafterweise um einen Aktivkohlefilter.

Die vorliegende Erfindung umfasst weiterhin ein Verfahren zur Herstellung von Reinstwasser in der Dialyse mittels einer Umkehr-Osmose-Anlage, wobei das Reinstwasser mit Ozon entkeimt wird. Dabei wird das Ozon durch einen stromabwärts der Umkehr-Osmose-Anlage angeordneten Ozon-Filter, insbesondere einen Aktivkohlefilter, entfernt. Durch dieses Verfahren ergeben sich offensichtlich die gleichen Vorteile, wie sie bereits oben hinsichtlich des Systems dargestellt wurden.

Vorteilhafterweise wird dabei das Verfahren mittels eines Systems durchgeführt, wie es oben dargestellt wurde, und/oder unter Verwendung einer Vorrichtung zur Herstellung von Reinstwasser in der Dialyse oder eines Dialysegeräts oder eines Dialysatschlauchsets oder Dialysefilters, wie sie oben dargestellt wurden.

Die vorliegende Erfindung wird nun anhand von Ausführungsbeispielen sowie einer Zeichnung näher beschrieben. Dabei zeigt:

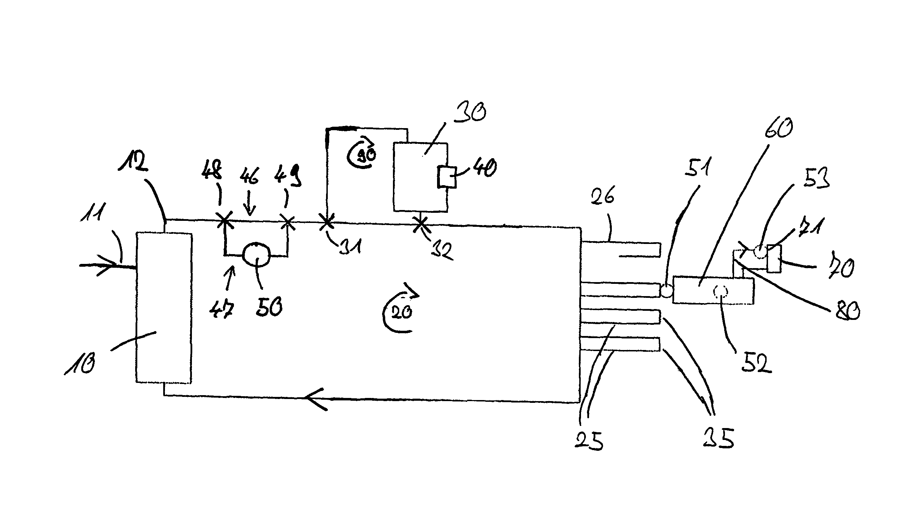

Figur 1: eine schematische Darstellung von Ausführungsbeispielen eines erfindungsgemäßen Systems.

In Figur 1 ist eine schematische Darstellung einer Vorrichtung zur Herstellung von Reinstwasser in der Dialyse gezeigt, wie sie im erfindungsgemäßen System zum Einsatz kommt. Die Vorrichtung umfasst dabei eine Umkehr-Osmose-Anlage 10, welche über einen Zulauf 11 zuströmendes Frischwasser reinigt und als Reinstwasser am Ablauf 12 zur Verfügung stellt. Dabei können im Bereich vor dem Zulauf der Umkehr-Osmose-Anlage selbstverständlich weitere Komponenten wie Vorfilter, Deionisierungsanlagen und ähnliches angeordnet sein, welche hier nicht näher dargestellt sind.

Die Vorrichtung zur Herstellung von Reinstwasser umfasst dabei einen Primärkreislauf 20 in Form einer Verteilungsringleitung, durch welche das von der Umkehr-Osmose-Anlage am Ablauf 12 zur Verfügung gestellte Reinstwasser zirkuliert. Im Primärkeislauf 20 kann dabei eine Pumpe vorgesehen sein, welche das Reinstwasser durch den Primärkreislauf zirkulieren lässt.

An diesem Primärkreislauf 20 sind mehrere Sekundärkreisläufe 25 angeschlossen, welche zu Anschlußstellen 35 für Dialysegeräte führen. Neben diesen Sekundärkreisläufen 25 ist weiterhin ein freier Auslauf 26 vorgesehen.

Neben der Vorrichtung zur Herstellung von Reinstwasser ist eine Dialysemaschine 60 schematisch dargestellt, welche an einem Sekundärkreislauf 25 der Vorrichtung zur Herstellung von Reinstwasser angeschlossen ist und Reinstwasser zur Herstellung von Dialysat bezieht. Die Herstellung des Dialysats durch Vermischung des Reinstwassers mit weiteren Komponenten in der Dialysemaschine ist in Figur 1 nicht näher dargestellt. Die Dialysemaschine 60 umfasst dabei einen Dialysator 70, welcher über Dialysatleitungen 80 mit dem Maschinenteil der Dialysemaschine verbunden ist. Sowohl der Dialysator als auch die Dialysatleitung bilden dabei üblicherweise Disposables, welche für eine Dialysebehandlung an den Maschinenteil angeschlossen werden. Während der Dialysebehandlung strömt dabei Dialysat durch die Dialysatleitungen zu einem Dialysateinlass 71 des Dialysators, durch diesen hindurch zu einem Dialysatauslass und von dort zurück zur Dialysemaschine.

Erfindungsgemäß ist nun eine Vorrichtung zur Erzeugung von Ozon vorgesehen, über welche der Reinstwasserbereich der Vorrichtung von Verkeimung freigehalten wird. Als Vorrichtung zur Erzeugung von Ozon wird im Ausführungsbeispiel eine elektrolytisch betriebene Anlage eingesetzt. Dabei wird ein Teilstrom des Wassers entnommen, in welchem durch Elektrolyse Sauerstoff und Wasserstoff erzeugt werden, wobei der Sauerstoff durch einen Katalysator in Ozon umgewandelt wird und im Wasser gelöst wird.

Bei dem in Figur 1 gezeigten Ausführungsbeispiel ist die Vorrichtung zum Einbringen von Ozon 30 dabei stromabwärts des Ablaufs 12 der Umkehr-Osmose-Anlage 10 angeordnet, das heißt das Ozon wird dem Reinstwasser zugesetzt, welches mit der Umkehr-Osmose-Anlage 10 erzeugt wird. Dabei ist die Vorrichtung zum Einbringen von Ozon im Primärkreislauf 20 angeordnet.

Der Ozonkreislauf 90 entnimmt dabei am Punkt 31 dem Primärkreislauf zumindest einen Teilstrom, in welchen das Ozon eingebracht wird. Zur Steuerung der Entnahme ist am Punkt 31 ein entsprechendes Ventil angeordnet. Der ozonisierte Teilstrom fließt dabei an der Stelle 32 wieder in den Primärkreislauf 20 und vermischt sich dort mit dem restlichen Reinstwasserstrom. Auch hier ist ein entsprechendes Ventil angeordnet. Nach dem Abschalten des Ozonkreislaufs (90) werden die Ventile (31) und (32) direkt verbunden. Die Vorrichtung zum Einbringen von Ozon kann daher komplett von der Hauptleitung des Primärkreislaufs getrennt werden.

Weiterhin ist ein Ozon-Filter vorgesehen, welcher Ozon-Restmengen wieder aus dem Reinstwasser entfernt und so eine Gefährdung des Dialysepatienten ausschließt. Als Ozon-Filter kann erfindungsgemäß ein Aktivkohlefilter eingesetzt werden. Insbesondere können dabei handelsübliche Aktivkohlefilter verwendet werden, da diese eine Ozon-Restmenge im Reinstwasser bzw. Dialysat ausreichend entfernen können.

Der Ozon-Filter kann dabei an unterschiedlichen Stellen des Wasserkreislaufs eingebaut werden. In Fig. 1 sind mit den Positionen 50 bis 53 vier unterschiedliche Möglichkeiten der Positionierung eines solchen Ozon-Filters beispielhaft dargestellt.

So kann der Ozon-Filter insbesondere im Primärkreislauf 20 angeordnet werden. Dies ist beispielhaft mit der Position 50 dargestellt. Der Primärkreislauf weist dabei im Bereich 50 zwei parallele Leitungen 46 und 47 auf, wobei der Reinstwasserstrom durch die Ventile 48 und 49 sowohl nur durch die Leitung 46, als auch nur durch die Leitung 47 geleitet werden kann. Der Ozon-Filter ist dabei in der Leitung 47 angeordnet, so dass der Reinstwasserstrom durch Ansteuerung der Ventile 48 und 49 durch den Ozon-Filter geleitet werden kann oder an diesem vorbei.

Bei einer Anordnung des Ozon-Filters im Primärkreislauf sollte dieser so positioniert werden, dass das in die Sekundärkreisläufe 25 bzw. zu den Dialysegeräten 60 hin fließende Reinstwasser bereits durch den Filter von Ozon befreit wurde. Der Filter ist daher in Umlaufrichtung zwischen dem Ablauf 12 der Umkehr-Osmose-Anlage und dem ersten Sekundärkreislauf 25 bzw. der ersten Anschlußstelle für Dialysemaschinen 35 positioniert werden, so dass den Dialysegeräten von Ozon befreites Reinstwasser zur Verfügung gestellt wird.

In Fig. 1 sind dabei die Vorrichtung zum Einbringen von Ozon und der Filter in diesem Bereich hintereinander angeordnet. Ozon-Filter können jedoch auch jeweils in den Sekundärkreisläufen 25 angeordnet werden, was in Figur 1 jedoch nicht näher dargestellt wurde.

Neben der Anordnung von Filtern im Bereich des Primär- oder der Sekundärkreisläufe ist es möglich, Ozon-Filter im Bereich der Anschlussstellen der Dialysegeräte 60 anzuordnen, was mit Position 51 dargestellt ist.

Dabei können sowohl im Primärkreislauf, als auch in den Sekundärkreisläufen und/oder den Anschlussstellen Ozon-Filter vorgesehen sein.

Weiterhin können auch die Dialysemaschinen 60 mit einem Ozon-Filter ausgestattet werden, was mit den Positionen 52 bzw. 53 dargestellt ist. Der Ozon-Filter kann dabei an geeigneter Stelle innerhalb des Maschinenteils der Dialysemaschine entweder im Bereich des Reinstwassers oder bereits im Bereich der Dialysierflüssigkeit angeordnet werden. Wie in Position 53 dargestellt, kann der Ozon-Filter auch in der Dialysatleitung 80 oder direkt am Dialysat-Zulauf 71 des Dialysators 70 angeordnet werden.

Wird der bzw. werden die Ozon-Filter im Bereich der Dialysegeräte angeordnet, kann an den Anschlüssen 35 der Vorrichtung zur Herstellung von Reinstwasser mit Ozon versetztes Reinstwasser zur Verfügung gestellt werden. Dies den Vorteil, dass das mit Ozon behandelte Reinstwasser den gesamten Reinstwasserbereich der Vorrichtung durchströmt und gegebenenfalls sogar noch Teile der Dialysemaschine. Eine Anordnung des Ozon-Filters im Bereich der Vorrichtung zur Herstellung des Reinstwassers selbst ist dagegen mit weniger Aufwand verbunden.

Die vorliegende Erfindung erlaubt es, das Reinstwasser in der Vorrichtung mit Ozon zu versetzen und so einer Verkeimung sicher vorzubeugen.

Dabei kann in regelmäßigen Abständen eine Ozonisierung des Reinstwasserbereiches durchgeführt werden, z.B. 1-5 mal pro Woche. Während der Ozonisierung fließt das Reinstwasser dabei vorteilhafterweise nicht durch die Leitung 47 mit dem Ozon-Filter, sondern ungefiltert durch die parallele Leitung 46, so dass der gesamte Reinstwasserbereich erreicht wird. Der Ozongenerator 40 liegt dabei in einem speziellen Ozonisierungskreislauf 90, um die Ozonisierung jederzeit stoppen zu können.

Nach Abschluss der Ozonisierung wird dann der Filter im Primärkreislauf aktiv geschaltet, indem der Reinstwasserstrom durch die Leitung 47 geleitet wird. So kann das Reinstwasser wieder sicher von Ozon befreit werden.

Die Vorrichtung umfasst dabei eine entsprechende Steuerung zur Ansteuerung der Vorrichtung 30 zur Einbringung von Ozon sowie der entsprechenden Ventile zur Ansteuerung der Flüssigkeitsströme.

Weiterhin können die Filter 51 bis 53 am Ende des Sekundärkreislauf permanent in die Anlage eingebracht sein, um ggf. übrig gebliebenes Ozon zu binden.

## Patentansprüche

1. System zur Herstellung von Reinstwasser in der Dialyse mit einer Umkehr-Osmose-Anlage (10), welche einen Zulauf (11) für Frischwasser und einen Ablauf (12) für Reinstwasser aufweist, und mit einer Vorrichtung (30) zum Einbringen von Ozon,
**dadurch gekennzeichnet,**
**dass** ein Ozon-Filter (50, 51, 52, 53) vorgesehen ist, welcher stromabwärts des Ablaufs (12) der Umkehr-Osmose-Anlage angeordnet ist, und eingebrachtes Ozon wieder entfernt.

2. System nach Anspruch 1, wobei es sich bei dem Ozon-Filter (50, 51, 52, 53) um einen Aktivkohlefilter handelt.

3. System nach Anspruch 1 oder 2, wobei die Vorrichtung (30) zum Einbringen von Ozon stromabwärts des Ablaufs (12) der Umkehr-Osmose-Anlage angeordnet ist und Ozon in das aus dem Ablauf der Umkehr-Osmose-Anlage fließende Reinstwasser einbringt.

4. System nach einem der vorangegangenen Ansprüche, wobei das System zur Herstellung von Reinstwasser eine Verteilungsringleitung (20) aufweist, in welcher Reinstwasser vom Ablauf der Umkehr-Osmose-Anlage aus an Anschlussbereichen für Dialysegeräte vorbei in einem Kreislauf gepumpt wird, wobei die Vorrichtung zum Einbringen von Ozon (30) in der Verteilungsringleitung angeordnet ist.

5. System nach einem der vorangegangenen Ansprüche, wobei das System zur Herstellung von Reinstwasser eine Verteilungsringleitung (20) aufweist, in welcher Reinstwasser vom Ablauf der Umkehr-Osmose-Anlage aus an Anschlussbereichen für Dialysegeräte vorbei in einem Kreislauf gepumpt wird, wobei der Ozon-Filter (50) in der Verteilungsringleitung angeordnet ist

6. System nach einem der vorangegangenen Ansprüche, wobei der Ozon-Filter (51) im Bereich eines zum Anschluss eines Dialysegerätes vorgesehenen Sekundärkreislaufs (25) angeordnet ist.

7. System nach Anspruch 1, 2 oder 3, wobei der Ozon-Filter (52, 53) im Bereich eines Dialysegerätes (60) angeordnet ist.

8. System nach Anspruch 7, wobei der Ozon-Filter (52, 53) im Dialysegerät in einem stromabwärts des Dialysateinlaufs des Dialysefilters (70) angeordneten Bereich oder direkt am Dialysateinlauf des Dialysefilters angeordnet ist.

9. System nach einem der vorangegangenen Ansprüche, mit einer Steuerung, welche die Vorrichtung zum Einbringen von Ozon so ansteuert, dass diese während des laufenden Betriebs des Systems Ozon erzeugt.

10. System nach einem der vorangegangenen Ansprüche, mit mehreren Anschlüssen (35) für Dialysegeräte (60), **dadurch gekennzeichnet, dass** das zum Betrieb der Dialysegeräte zur Verfügung gestellte Reinstwasser Ozon enthält.

11. System nach einem der vorangegangenen Ansprüche umfassend ein Dialysegerät (60) mit einem Anschluss zur Verbindung mit einer Vorrichtung zur Herstellung von Reinstwasser, wobei der Ozonfilter (51, 52, 53) im Bereich des Anschlusses, in einem Bereich stromabwärts des Anschlusses und stromaufwärts eines Dialysateinlaufs eines Dialysefilters (70) oder direkt am Dialysateinlauf des Dialysefilters (70) angeordnet ist.

12. System nach einem der vorangegangenen Ansprüche mit einem Dialysefilter (70), wobei der Ozon-Filter (52, 53) in einem stromaufwärts des Dialysateinlaufs des Dialysefilters (70) angeordneten Bereich oder direkt am Dialysateinlauf des Dialysefilters (70) angeordnet ist.

13. System nach einem der vorangegangenen Ansprüche mit einem Dialysatschlauchset (80) und einem Dialysefilter (70), wobei der Ozon-Filter (52, 53) in einem stromaufwärts des Dialysateinlaufs des Dialysefilters (70) angeordneten Bereich oder direkt am Dialysateinlauf des Dialysefilters (70) angeordnet ist.

14. Verfahren zur Herstellung von Reinstwasser in der Dialyse mittels einer Umkehr-Osmose-Anlage (10), wobei das Reinstwasser mit Ozon entkeimt wird und wobei das Ozon durch einen stromabwärts der Umkehr-Osmose-Anlage angeordneten Ozon-Filter (50, 51, 52, 53) entfernt wird.

15. Verfahren nach Anspruch 14 unter Verwendung eines Systems nach einem der vorangegangenen Ansprüche.

## Claims

1. System for producing ultra-pure water in the dialysis with a reverse osmosis plant (10) which includes in inlet (11) for fresh water and an outlet (12) for ultra-pure water, and with a device (30) for introducing ozone,
**characterized in**
**that** an ozone filter (50, 51, 52, 53) is provided, which is arranged downstream of the outlet (12) of the reverse osmosis plant and again removes introduced ozone.

2. The system according to claim 1, wherein the ozone filter (50, 51, 52, 53) is an activated carbon filter.

3. The system according to claim 1 or 2, wherein the device (30) for introducing ozone is arranged downstream of the outlet (12) of the reverse osmosis plant and introduces ozone into the ultra-pure water flowing out of the outlet of the reverse osmosis plant.

4. The system according to any of the preceding claims, wherein the system for producing ultra-pure water includes a distribution ring line (20) in which ultrapure water is pumped in a circuit from the outlet of the reverse osmosis plant past connecting regions for dialysis machines, wherein the device for introducing ozone (30) is arranged in the distribution ring line.

5. The system according to any of the preceding claims, wherein the system for producing ultra-pure water includes a distribution ring line (20) in which ultra-pure water is pumped in a circuit from the outlet of the reverse osmosis plant past connecting regions for dialysis machines, wherein the ozone filter (50) is arranged in the distribution ring line.

6. The system according to any of the preceding claims, wherein the ozone filter (51) is arranged in the region of a secondary circuit (25) provided for the connection of a dialysis machine.

7. The system according to claim 1, 2 or 3, wherein the ozone filter (52, 53) is arranged in the region of a dialysis machine (60).

8. The system according to claim 7, wherein the ozone filter (52, 53) in the dialysis machine is arranged in a region downstream of the dialysate inlet of the dialysis filter (70) or directly at the dialysate inlet of the dialysis filter.

9. The system according to any of the preceding claims, with a controller which actuates the device for introducing ozone such that the same generates ozone during the operation of the system.

10. The system according to any of the preceding claims, with several ports (35) for dialysis machines (60), **characterized in that** the ultra-pure water provided for the operation of the dialysis machines contains ozone.

11. The system according to any of the preceding claims, comprising a dialysis machine (60) with a port for connection with an apparatus for producing ultra-pure water, wherein the ozone filter (51, 52, 53) is arranged in the region of the port, in a region downstream of the port and upstream of a dialysate inlet of a dialysis filter (70) or directly at the dialysate inlet of the dialysis filter (70).

12. The system according to any of the preceding claims with a dialysis filter (70), wherein the ozone filter (52, 53) is arranged in a region arranged upstream of the dialysate inlet of the dialysis filter (70) or directly at the dialysate inlet of the dialysis filter (70).

13. The system according to any of the preceding claims with a dialysate tube set (80) and a dialysis filter (70), wherein the ozone filter (52, 53) is arranged in a region upstream of the dialysate inlet of the dialysis filter (70) or directly at the dialysate inlet of the dialysis filter (70).

14. A method for producing ultra-pure water in the dialysis by means of a reverse osmosis plant (10), wherein the ultra-pure water is sterilized with ozone and wherein the ozone is removed by an ozone filter (50, 51, 52, 53) arranged downstream of the reverse osmosis plant.

15. The method according to claim 14 by using a system according to any of the preceding claims.

## Revendications

1. Système de production d'eau ultrapure en dialyse comportant une installation d'osmose inverse (10), présentant une alimentation (11) en eau fraîche et une évacuation (12) d'eau ultrapure, et comportant un dispositif (30) pour l'introduction d'ozone,
**caractérisé en ce que**
un filtre à ozone (50, 51, 52, 53) est prévu, disposé en aval de l'évacuation (12) de l'installation d'osmose inverse et éliminant à nouveau l'ozone introduite.

2. Système selon la revendication 1, dans lequel le filtre à ozone (50, 51, 52, 53) est un filtre à charbon actif.

3. Système selon la revendication 1 ou 2, dans lequel le dispositif (30) pour l'introduction d'ozone est disposé en aval de l'évacuation (12) de l'installation d'osmose inverse et introduit de l'ozone dans l'eau ultrapure s'écoulant depuis l'évacuation de l'installation d'osmose inverse.

4. Système selon l'une des revendications précédentes, dans lequel le système pour la production d'eau ultrapure présente une conduite de distribution annulaire (20), dans laquelle de l'eau ultrapure est pompée dans un circuit depuis l'évacuation de l'installation d'osmose inverse en passant par des zones de raccordement pour des appareils de dialyse, le dispositif pour l'introduction d'ozone (30) étant disposé dans la conduite de distribution annulaire.

5. Système selon l'une des revendications précédentes, dans lequel le système pour la production d'eau ultrapure présente une conduite de distribution annulaire (20), dans laquelle de l'eau ultrapure est pompée dans un circuit depuis l'évacuation de l'installation d'osmose inverse en passant par des zones de raccordement pour des appareils de dialyse, le filtre à ozone (50) étant disposé dans la conduite de distribution annulaire.

6. Système selon l'une des revendications précédentes, dans lequel le filtre à ozone (51) est disposé dans la zone d'un circuit secondaire (25) prévu pour le raccordement d'un appareil de dialyse.

7. Système selon la revendication 1, 2 ou 3, dans lequel le filtre à ozone (52, 53) est disposé dans la zone d'un appareil de dialyse (60).

8. Système selon la revendication 7, dans lequel le filtre à ozone (52, 53) dans l'appareil de dialyse est disposé dans une zone en aval de l'entrée de dialysat du filtre de dialyse (70) ou directement au niveau de l'entrée de dialysat du filtre de dialyse.

9. Système selon l'une des revendications précédentes, comportant une commande, qui pilote le dispositif pour l'introduction d'ozone de telle manière que ce dernier produit de l'ozone en cours de fonctionnement du système.

10. Système selon l'une des revendications précédentes, comportant plusieurs raccordements (35) pour des appareils de dialyse (60), **caractérisé en ce que** l'eau ultrapure mise à la disposition pour le fonctionnement des appareils de dialyse contient de l'ozone.

11. Système selon l'une des revendications précédentes, comportant un appareil de dialyse (60) avec un raccordement de liaison à un dispositif de production d'eau ultrapure, le filtre à ozone (51, 52, 53) étant disposé dans la zone du raccordement, dans une zone en aval du raccordement et en amont d'une entrée de dialysat d'un filtre de dialyse (70) ou directement au niveau de l'entrée de dialysat du filtre de dialyse (70).

12. Système selon l'une des revendications précédentes, comportant un filtre de dialyse (70), le filtre à ozone (52, 53) étant disposé dans une zone en amont de l'entrée de dialysat du filtre de dialyse (70) ou directement au niveau de l'entrée de dialysat du filtre de dialyse (70).

13. Système selon l'une des revendications précédentes, comportant un ensemble de tubes de dialysat (80) et un filtre de dialyse (70), le filtre à ozone (52, 53) étant disposé dans une zone en amont de l'entrée de dialysat du filtre de dialyse (70) ou directement au niveau de l'entrée de dialysat du filtre de dialyse (70).

14. Procédé de production d'eau ultrapure en dialyse au moyen d'une installation d'osmose inverse (10), dans lequel l'eau ultrapure est stérilisée à l'ozone et dans lequel l'ozone est retiré par un filtre à ozone (50, 51, 52, 53) disposé en aval de l'installation d'osmose inverse.

15. Procédé selon la revendication 14 utilisant un système selon l'une des revendications précédentes.
